# EUROPEAN PATENT APPLICATION

(11) **EP 4 324 451 A1**
(43) Date of publication of application: **21.02.2024**
(21) Application number: 22787175.3
(22) Date of filing: 12.04.2022
(51) Int. Cl.: A61K 8/18, A61K 8/362, A61K 8/64, A61K 8/60, A61Q 5/00, A61Q 5/04

(54) **HAIR COMPOSITIONS, HAIR FORMULATIONS, PROCESSES OF PRODUCTION THEREOF, USES AND METHODS OF HAIR TREATMENT**

(30) Priority: 12.04.2021 BR 102021006988
(71) Applicant: Chemyunion Ltda, 18087-101 Sorocaba (BR)
(72) Inventor: PRINCIVAL, Cleverson Rogério, 18016-085 Sorocaba (BR); ROSSAN, Marcos Roberto, 18103-185 Sorocaba (BR); MUSSI, Lilian, 18013-240 Sorocaba (BR); JUNIOR, Flávio Bueno De Camargo, 18013-004 Avenida São Paulo, 1791, Além Ponte (BR); MAGALHÃES, Wagner Vidal, 18090-360 Sorocaba (BR); CAMILLO, Andrew Thomaz, 18025-176 Sorocaba (BR)
(74) Representative: Grünecker Patent- und Rechtsanwälte PartG mbB
(86) International application number: PCT/BR2022/050130
(87) International publication number: WO 2022/217330

(57) **Abstract**

The invention is a hair composition that acts in the restoration and strengthening of hair, production processes of said composition, its uses and hair treatment methods. More specifically, the invention comprises the use of bifunctional α,β-unsaturated compounds and their derivatives in combination with ionic and non-ionic crosslinking agents to promote the formation of crosslinks between the polypeptide chains of keratin through ionic interactions, hydrogen bonds, and also by C-N type covalent bonds. The present invention lies in the fields of Chemistry, Biochemistry and Pharmacy.

## Description

### Field of the Invention

The invention refers to hair compositions that act in the restoration and strengthening of hair, comprising bifunctional α,β-unsaturated compounds associated with ionic and non-ionic crosslinking agents, and the present invention discloses production processes of said composition, its uses and methods of hair treatment. The present invention lies in the fields of Chemistry, Biochemistry and Pharmacy.

### Background of the Invention

Hair is constantly damaged through washing, thermal treatments, chemical transformation treatments, such as straightening and, mainly, by bleaching and dyeing processes. These procedures most of the time lead to changes in the protein structure of keratin, leading to the breaking of covalent bonds present in the hair structure, making the hair fragile and brittle.

Currently, the process of bleaching and dyeing hair is a common activity, regardless of age, ethnicity, social class or gender. In this process, the hair is chemically attacked by oxidizing and root agents, which can compromise the structure of the hair fiber because, in addition to the degradation of the hair fiber pigments, eumelanin and pheomelanin, lateral reactions of protein oxidation occur.

Patent document US2020315936 entitled "Hair strengthening ingredient and method for strengthening hair" discloses hair compositions comprising bifunctional acids and bases that increase hair strength by intermolecular interactions without changing the structure of keratin, acting in the pH range of 1.5 to 7. The document does not disclose the combinations of α,β-unsaturated compounds with the crosslinking agents as defined in the present patent application. Further, the document mentions results only on hair without chemical treatments.

Patent document BR112016002255 entitled "Cosmetic method for treating hair, formulation, kits and shampoo or conditioner" refers to a cosmetic method for treating hair based on repairing disulfide bonds in hair or skin, revealing compounds that interact with keratin and that are capable of reacting with one or more thiol groups.

Patent document US2021/0046334(A1) entitled "Methods for fixing hair and skin" refers to a hair composition to strengthen hair comprising an ionic compound that may comprise itaconate groups, but does not disclose a combination of crosslinking agents as defined in the present patent application. Document US2021/0046334(A1) claims protection for a composition containing a biding agent (formed by an ionic compound) comprising a linker ionically linked to at least two other reactive groups ("reactive moieties"), wherein the itaconoate group can be selected as a reactive group.

Thus, depending on the researched literature, no documents were found anticipating or suggesting the teachings of the present invention, so that the formulation proposed herein has novelty and inventive activity when compared to the state of the art.

In short, it is possible to observe there is still a need to develop more efficient hair compositions, especially for application on hair weakened by chemical treatments.

### Summary of the Invention

Thus, the present invention solves the problems of the state of the art through the development of compositions comprising bifunctional α,β-unsaturated compounds associated with ionic and non-ionic crosslinking agents. These compositions are capable of reacting with amino groups present in the protein structure of keratin through reactions of nucleophilic and/or root addition of amino groups to compounds, strengthening and maintaining the shine of hair strands. Furthermore, the formulation may also comprise actives capable of catalyzing disulfide bonds between the keratins present in the hair.

In a first object, the present invention features a hair composition comprising:
i. from 0.1% to 30% by mass of at least one bifunctional α,β-unsaturated compound and its derivatives;
ii. from 2% to 60% by mass of crosslinking agent, wherein:
   a) from 1 % to 40% by mass is of a non-ionic crosslinking agent selected from the group consisting of polyols, saccharides, or a combination thereof;
   b) from 1% to 20% by mass is of an ionic crosslinking agent selected from the group consisting of hydrolyzed proteins, amino acids, or a combination thereof;
iii. water q.s. 100%.

In a second object, the present invention features a hair composition production process, as defined in the present patent application, comprising the following step:
a) mix from 0.1% to 30% by mass of at least one bifunctional α,β-unsaturated compound with 2% to 60% by mass of at least one crosslinking agent and water q.s. 100%.

In a third object, the present invention discloses the use of a hair composition, as defined in the present patent application, in product formulations, such as: conditioners, leave-in, masks, serum, creams, shampoos, sprays, lotions, and gels.

In a fourth object, the present invention features a hair formulation comprising from 0.1% to 30% by mass of hair composition as defined in the present patent application and at least one cosmetically and/or pharmaceutically acceptable excipient.

In a fifth object, the present invention discloses a hair treatment method comprising the application to the hair of a hair composition as defined in the present patent application.

These and other objects of the invention will be immediately appreciated by those skilled in the art and will be described in detail below.

### Brief Description of the Figures

The following figures are presented:

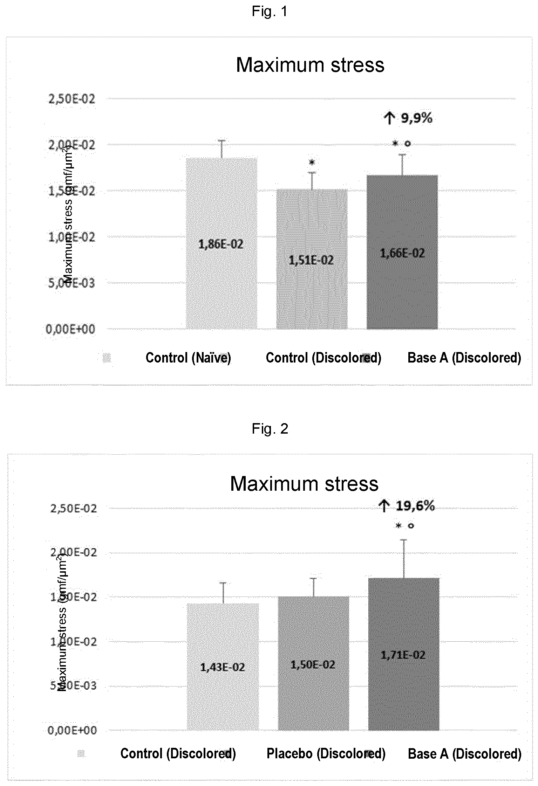

Figure 1 shows the results referring to the protective effect during 3 discoloration processes in relation to the maximum tension promoted by the active, Base A. Where "*" indicates the significant increase in relation to the Control group (Naive) (p<0.001; p<0.01) and "°" indicates the significant increase in relation to the Control group (Discolored) (p<0.01).
Figure 2 shows the results referring to the repairing effect in relation to the maximum tension after 3 discoloration processes, applying Base A. Where "*" indicates the significant increase in relation to the Control group (p<0.01) and "°" indicates the significant increase compared to the Placebo group (p<0.05).

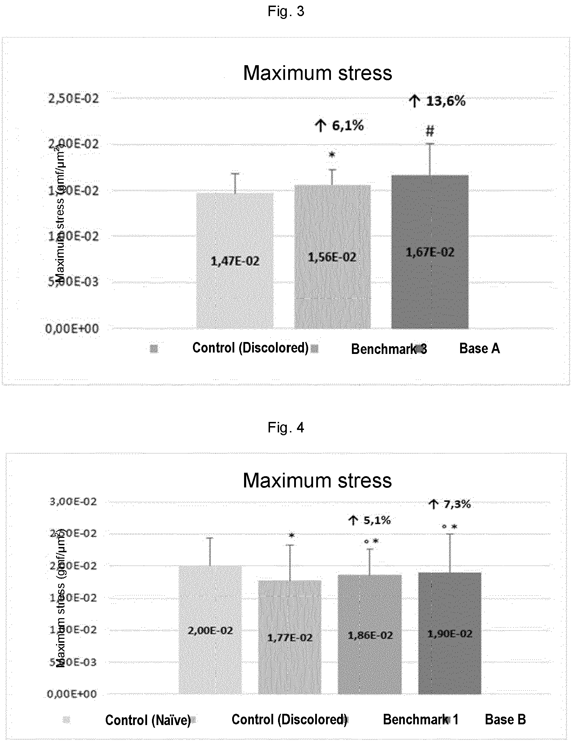

Figure 3 shows the results referring to the repairing effect in relation to the maximum tension after 3 discoloration processes, applying Base A 5% in a leave-in base, and comparing it with Benchmark 3, which consists in a ready-to-use cosmetic base. Where "*" indicates the significant difference compared to the Control (Discolored) (p=0.02) and "#" indicates the significant difference compared to the Control (Discolored) (p=0.00015).
Figure 4 shows the results regarding the protective effect during 2 discoloration processes with respect to the maximum tension promoted by Base B compared to Benchmark 1. With "*" indicates the significant increase in relation to the Discolored control group (p<0.05), "°" indicates the significant reduction in relation to the Naive control group (p<0.05).

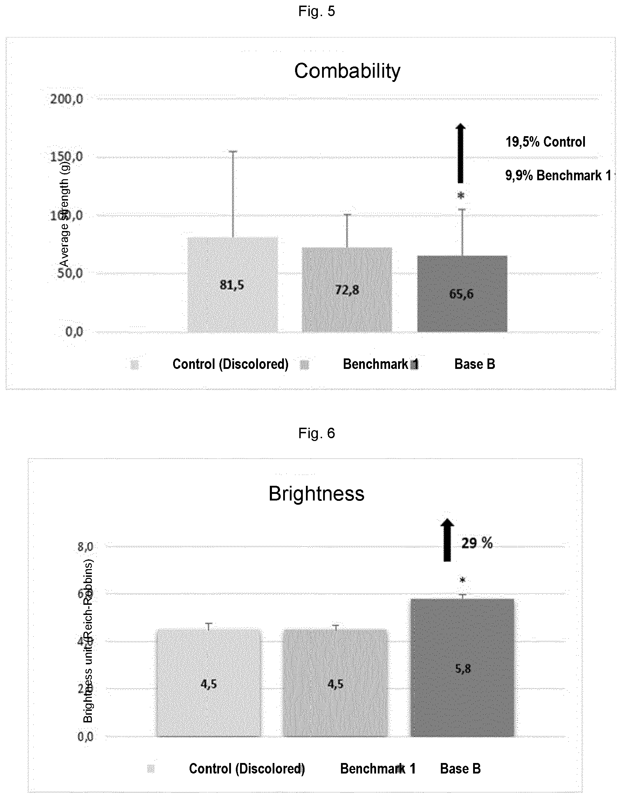

Figure 5 shows the results regarding the protective effect during 2 discoloration processes with respect to the maximum combing force promoted by Base B compared to Benchmark 1. Where "*" indicates the significant reduction in relation to the group Benchmark 1 (p<0.05).
Figure 6 shows the results regarding the protective effect during 2 discoloration processes with respect to the brightness promoted by Base B compared to Benchmark 1. Where "*" indicates the significant increase in relation to the Control and Benchmark groups 1 (p<0.001).

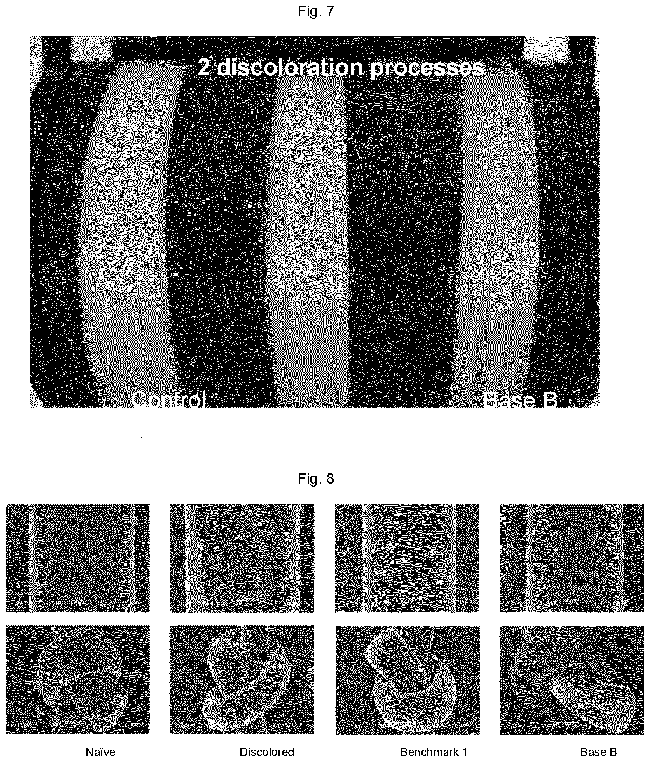

Figure 7 shows the hair strands after the protective effect during 2 discoloration processes with respect to the brightness promoted by Base B compared to Benchmark 1.
Figure 8 shows the results regarding the protective effect during 2 discoloration processes regarding the protection of the cuticular surface by Scanning Electron Microscopy (SEM) promoted by Base B and compared to Benchmark 1. In this study, 5 capillary fibers per group (2 images per fiber) were evaluated with magnification from 400 to 1100 times.

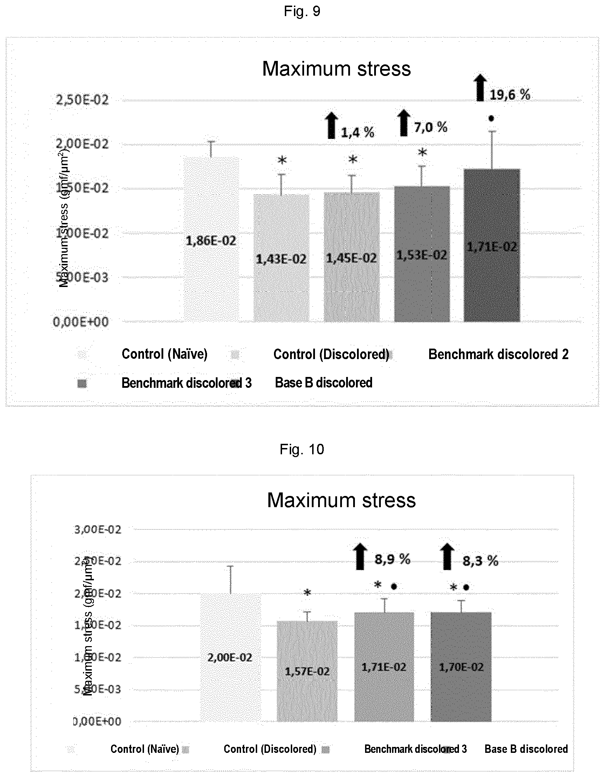

Figure 9 shows the results regarding the repairing effect in relation to the maximum tension after 3 discoloration processes applying Base B at 5% in a leave-in basis, and comparing it with Benchmarks 2 and 3, which consist of ready-to-use cosmetic bases. Where "*" indicates a significant reduction in relation to the naive control (p<0.001) and "•" indicates a significant increase in relation to the discolored control and Benchmark 2 groups (p<0.01 and p<0.05).
Figure 10 shows the results regarding the repairing effect, after 3 discoloration processes, in relation to the maximum tension, applying Base B at 1% in a leave-in basis, and comparing it with Benchmark 3, which consists of a ready-to-use cosmetic base. Where "*" indicates a significant reduction in relation to the naive control (p<0.001) and "•" indicates a significant increase in relation to the discolored control group (p<0.01).

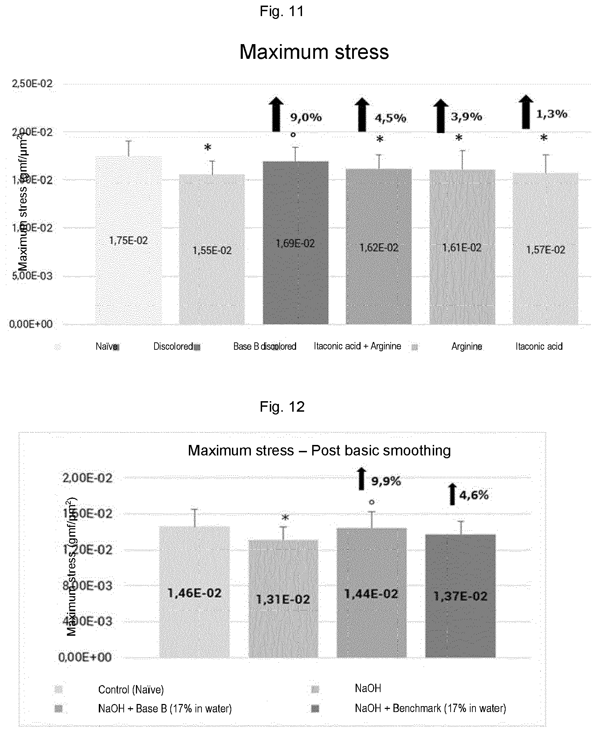

Figure 11 shows the results referring to the repairing effect after 3 discoloration processes in relation to the maximum tension, showing the synergy of the components of Base B, applying Base B at 5% in a leave-in base, as well as each of the ingredients of Base B in a leave-in base. Since "*" indicates a significant reduction in relation to the naive group (p<0.001; p<0.01; p<0.01; p<0.001), "°" indicates a significant reduction in relation to the group treated with Base B (p<0.001; p<0.01; p<0.01; p<0.001).
Figure 12 shows the results of the repairing effect after basic straightening with alkaline straightener (NaOH) in relation to maximum tension tests, applying after the time of action of the basic straightener, Base B and Benchmark 1 in a 17% aqueous solution for 5 minutes. Where "*" indicates a significant reduction in relation to the control group (p<0.001) and "°" indicates a significant increase in relation to the group treated with NaOH (p<0.001; p<0.01).

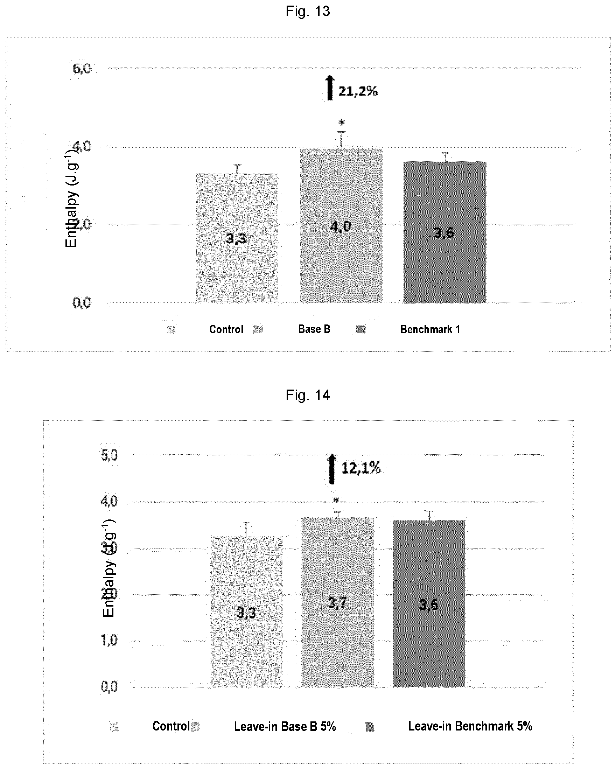

Figure 13 shows the results of the protective effect of the alpha-keratin structure during 3 bleaching processes with respect to enthalpy measurements by DSC (Differential Scanning Calorimetry) promoted by Base B compared to Benchmark 1. Where "*" indicates a significant increase in relation to the discolored control group (p<0.05).
Figure 14 shows the results of the repairing effect of alpha-keratin structure after 3 bleaching processes with respect to enthalpy measurements by DSC (Differential Scanning Calorimetry) promoted by Base B compared to Benchmark 1. Where "*" indicates a significant increase in relation to the discolored control group (p<0.05).

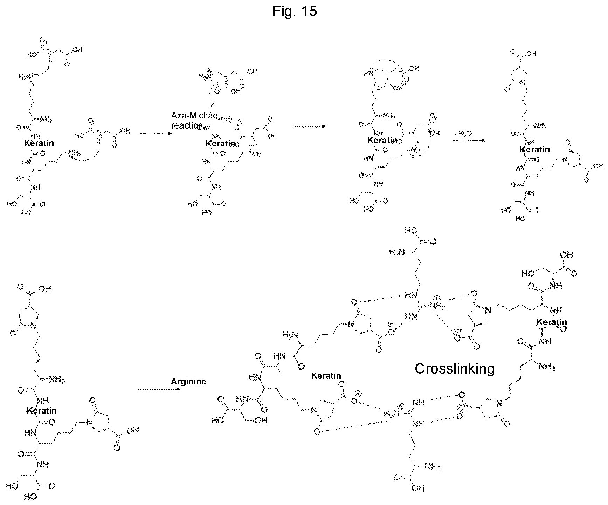

Figure 15 is a representation of the mechanism for the Aza-Michael reaction, between itaconic acid and amino group residues present in keratin.

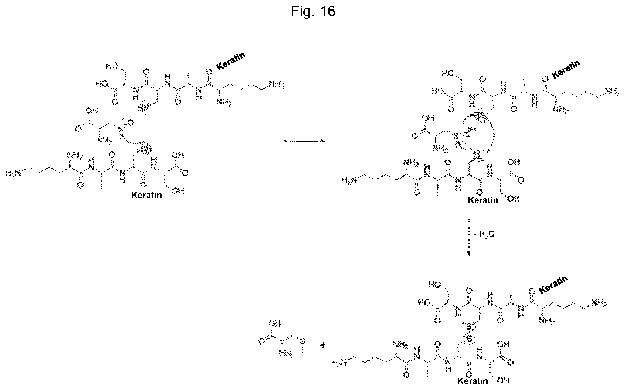

Figure 16 indicates the mechanism of action of sulfoxide-catalyzed disulfide bond.

### Detailed Description of the Invention

The present invention features hair compositions that act in the restoration and strengthening of hair; the present invention also discloses the production processes of said compositions, uses thereof and methods of hair treatment. The actives of the present composition act synergistically in increasing resistance, as well as act in moisture retention, thickening, brightness, softness, thermal protection, sealing the cuticles and improving the sensorial properties of the hair.

In the prior art, compositions for hair strengthening comprising bifunctional α,β-unsaturated compounds, for example, dicarboxylic acids, are already known. However, these compositions still do not show satisfactory results, especially after hair methods that damage the hair, such as discoloration and dyeing. In this way, several studies are being carried out to enhance the strengthening effect of these compositions.

The main hair strengthening compositions comprising bifunctional α,β-unsaturated compounds add crosslinking compounds in order to increase the promotion of crosslink formation. In the present invention, we present a composition that comprises the promotion of hair strengthening by promoting the formation of crosslinks from bifunctional α,β-unsaturated compounds in combination with ionic and non-ionic crosslinking agents, which, in addition to promoting the crosslinking, strengthens these interactions.

More specifically, the present invention features a synergistic composition of bifunctional α,β-unsaturated compounds with non-ionic crosslinking agents selected from the group consisting of polyols, saccharides, or combination thereof, and ionic crosslinking agents, selected from the group consisting of hydrolyzed proteins, amino acids, or a combination thereof. In one embodiment, the composition has a synergistic effect of itaconic acid and its derivatives with pro-vitamin B5, saccharides and/or polysaccharides, and hydrolyzed proteins.

These combinations of crosslinking agents mentioned above with bifunctional α,β-unsaturated compounds promote a surprisingly high formation of crosslinks, increasing hair strengthening when compared to other compositions of bifunctional α,β-unsaturated compounds with common crosslinking agents, and still promotes brightness, combability and softness of hair strands. Thus, the hair compositions of the present patent application show properties that are much superior to the use of separate actives, or the simple combination of bifunctional α,β-unsaturated compounds with common crosslinking agents.

Additionally, the composition may comprise methionine sulfoxide which repairs sulfur bonds in the hair (cysteine). Another advantage of methionine sulfoxide in hair composition stems from the fact that the methionine sulfoxide molecule, in its hydrochloride form, is soluble in aqueous systems, facilitating its incorporation into any cosmetic formulation, being compatible with most commonly used ingredients, with the exception of molecules containing sulfhydryl group (-SH), which can react with methionine sulfoxide leading to the formation of the corresponding disulfide.

In general, the formulation was developed to reduce damage caused by hair stress from chemical and thermal processes, considering three elementary properties.

The first property is related to the reactions between unsaturated, synthetic and/or natural compounds with the amino group present in the keratin structure.

The second property contemplates the formation of crosslinks between the polypeptide chains of keratin through ionic interactions, hydrogen bonds, and also by C-N type covalent bonds.

The third property, optional, is related to the cystine formation reactions that are formed by the bonds between cysteine residues catalyzed by sulfoxide derivatives, among them methionine sulfoxide.

Thus, in one embodiment, a product was developed capable of increasing hair strength through reactions with an amino group contained in the side chains of amino acids present in the keratin structure that react via Michael and Aza-Michael reactions, with derivatives of α,β-unsaturated compounds, leading to the formation of tertiary amines, and in one embodiment, using the concept of restoring disulfide bonds. Furthermore, the product of this reaction strongly interacts with crosslinking agents, such as arginine, low molecular weight hydrolyzed proteins and the different amino acid residues present in the protein structure of the hair. And surprisingly, the inclusion of non-ionic crosslinking agents, such as disaccharides, pro-vitamin B5 and polysaccharides showed a synergistic association forming crosslinks between the polypeptide chains of keratin.

Also, the actives of the hair compositions of the present patent application can be obtained by biotechnological processes and from renewable vegetable sources.

In short, hair compositions are disclosed herein with surprising effects compared to the prior art, mainly due to the substantial increase in relation to the mechanical properties, brightness and sensation (softness) in relation to other compositions comprising bifunctional α,β-unsaturated compounds.

In one embodiment, such compositions proved capable of promoting hair strengthening by different mechanisms of action, since methionine sulfoxide catalyzes the formation of disulfide bonds and, in parallel, the bifunctional α,β-unsaturated compounds make the formation of two Aza-Michael type covalent bonds possible, leading to modification of the keratin polypeptide chains. In addition, the formation of crosslinks can occur through amino acids or hydrolyzed proteins, through intermolecular interactions, forming "bridges" or "ionic crosslinks" with these modified polypeptide chains, promoting expressive increases in the maximum breaking tension of discolored hair. Furthermore, the increase in strength can be enhanced by associating it with pro-vitamin B5, saccharides and polysaccharides.

In a first object, the present invention features a hair composition comprising:
i. from 0.1% to 30% by mass of at least one bifunctional α,β-unsaturated compound and its derivatives;
ii. from 2% to 60% by mass of crosslinking agent, wherein:
   a) from 1% to 40% by mass of the hair composition is a non-ionic crosslinking agent selected from the group consisting of polyols, saccharides, or a combination thereof;
   b) from 1% to 20% by mass of the hair composition is an ionic crosslinking agent selected from the group consisting of hydrolyzed proteins, amino acids, or a combination thereof;
iii. water q.s. 100%.

In one embodiment, the hair composition comprises from 0.1% to 50% by mass of methionine sulfoxide. In one embodiment, the hair composition comprises from 0.1% to 40% by mass of methionine sulfoxide. In one embodiment, the hair composition comprises from 0.1% to 30% by mass of methionine sulfoxide. In one embodiment, the hair composition comprises from 0.1% to 20% by mass of methionine sulfoxide. In one embodiment, the hair composition comprises from 0.1% to 10% by mass of methionine sulfoxide. In one embodiment, the hair composition comprises from 10% to 50% by mass of methionine sulfoxide. In one embodiment, the hair composition comprises from 20% to 50% by mass of methionine sulfoxide. In one embodiment, the hair composition comprises from 30% to 50% by mass of methionine sulfoxide. In one embodiment, the hair composition comprises from 40% to 50% by mass of methionine sulfoxide. In one embodiment, the hair composition comprises from 10% to 40% by mass of methionine sulfoxide. In one embodiment, the hair composition comprises from 20% to 40% by mass of methionine sulfoxide. In one embodiment, the hair composition comprises from 30% to 40% by mass of methionine sulfoxide.

In one embodiment, the composition comprises from 1% to 30% of an α,β-unsaturated compound. In one embodiment, the composition comprises from 1% to 25% of an α,β-unsaturated compound. In one embodiment, the composition comprises from 2% to 25% of an α,β-unsaturated compound. In one embodiment, the composition comprises from 1% to 20% of an α,β-unsaturated compound. In one embodiment, the composition comprises from 5% to 30% of an α,β-unsaturated compound. In one embodiment, the composition comprises from 5% to 20% of an α,β-unsaturated compound.

In one embodiment, the α,β-unsaturated compound is an α,β-unsaturated dicarboxylic acid or ester. In one embodiment, the α,β-unsaturated compound is derived from itaconic acid, acrylic acid, maleic acid, fumaric acid, methacrylic acid and/or maleic anhydride. In one embodiment, the α,β-unsaturated compound is a polyol diester. In one embodiment, the α,β-unsaturated compound is panthenol diitaconate. In one embodiment, the α,β-unsaturated compound is obtained from a fermented *Aspergillus* extract.

In one embodiment, the composition comprises from 5% to 20% by mass of an ionic crosslinking agent. In one embodiment, the composition comprises from 10% to 20% by mass of an ionic crosslinking agent.

In one embodiment, the composition comprises from 1% to 20% by mass of amino acids. In one embodiment, the composition comprises from 5% to 20% by mass of amino acids. In one embodiment, the amino acid is arginine.

In one embodiment, the composition comprises from 0.1% to 10% by mass of hydrolyzed proteins. In one embodiment, the composition comprises from 1% to 10% by mass of hydrolyzed proteins. In one embodiment, the hydrolyzed proteins are rice and pea proteins.

In one embodiment, the composition comprises from 3% to 40% by mass of a non-ionic crosslinking agent. In one embodiment, the composition comprises from 3% to 35% by mass of a non-ionic crosslinking agent. In one embodiment, the composition comprises from 5% to 20% by mass of a non-ionic crosslinking agent. In one embodiment, the composition comprises from 1% to 15% by mass of a non-ionic crosslinking agent.

In one embodiment, the composition comprises from 0.1% to 20% by mass of polyols. In one embodiment, the composition comprises from 1% to 15% by mass of polyols. In one embodiment, the polyols are selected from panthenol and 1,3-propanediol.

In one embodiment, the composition comprises from 0.1% to 30% by mass of saccharides. In one embodiment, the composition comprises from 1% to 20% by mass of saccharides. In one embodiment, the saccharides are polysaccharides and/or disaccharides. In one embodiment, the saccharides are of plant origin. In one embodiment, the saccharide is trehalose. In one embodiment, the saccharides are obtained from a linseed or chia extract. In one embodiment, linseed means the species and cultivars of *Linum spp.* In one embodiment, chia means the species and cultivars of *Salvia spp.*

It is understood in the present invention that the term "derivatives" comprises their hydrates, stereoisomers, multimers, cyclic forms, drugconjugates, pro-drug and their cosmetically or pharmaceutically acceptable salts. Also, "derivatives" of α,β-unsaturated compounds include organic derivatives, such as esters, amides, hydroxys, ethers, amines, among other common organic functions.

It is understood in the present invention that the % by mass refers to the total composition and/or the total formulation.

In one embodiment, the hair composition comprises from 1% to 15% by mass of additives from the hair composition. In one embodiment, additives are selected from the group consisting of preservatives, antimicrobial agents, emulsifiers, solvents and humectants.

In one embodiment, the hair composition comprises:
i. from 5% to 20% by mass of at least one bifunctional α,β-unsaturated compound selected from the group consisting of itaconic acid and its derivatives;
ii. from 6% to 55% by mass of crosslinking agent, wherein:
   a) from 1% to 35% by mass of the hair composition is of a non-ionic crosslinking agent selected from the group consisting of polyols, saccharides, or combinations thereof;
   b) from 5% to 20% by mass is arginine;
iii. water q.s. 100%.

In a second object, the present invention features a hair composition production process, as defined in the first object, comprising the following step:
a) mix from 0.1% to 30% by mass of at least one bifunctional α,β-unsaturated compound, with 2% to 60% by mass of at least one crosslinking agent and water q.s. 100%.

In a third object, the present invention discloses the use of a hair composition, as defined in the first object, in product formulations, such as: conditioners, leave-in, masks, serum, creams, shampoos, sprays, lotions, and gels.

In a fourth object, the formulation of the products comprises from 0.1% to 30% by mass of said hair composition and at least one cosmetically and/or pharmaceutically acceptable excipient. In one embodiment, the product formulation comprises from 0.5% to 30% by mass of said hair composition. In one embodiment, the formulation comprises from 1% to 30% by mass of said hair composition. In one embodiment, the formulation comprises from 1% to 20% by mass of said hair composition. In one embodiment, the formulation comprises from 1% to 10% by mass of said hair composition.

In one embodiment, the formulation is cosmetically or pharmaceutically suitable for human and/or veterinary use. An adequate cosmetic and pharmaceutical formulation means any and all types of solvents, dispersion media, encapsulations, permeation enhancing agents, surfactants, preservatives and other ingredients, alone or in combination, and which are physiologically compatible, i.e., when applied do not present instability, incompatibility, toxicity and do not cause allergic reactions or irritability.

In one embodiment, the formulation comprises at least one pharmaceutically and/or cosmetically acceptable excipient selected from the group consisting of solubilizers, preservatives, surfactants, carriers, colorants, antioxidants, fillers, surfactants, UVA and/or UVB filters, fragrances, thickeners, humectants, whitening agents, anti-mycotic agents, anti-parasitic agents, antimicrobial agents, external analgesics, sunscreens, photoprotectors, keratolytic agents, detergents/surfactants, moisturizers, nutrients, vitamins, energy enhancers, anti-perspiration agents, astringents, deodorants, hair removers, firming agents, anti-scaling agents, anti-static agents. In one embodiment, the pharmaceutically and/or cosmetically acceptable excipient is selected from the group consisting of emulsifier, preservative and solubilizer. Examples of cosmetically acceptable ingredients and/or excipients can be found in handbooks and pharmacopoeias of each country. Non-exhaustive examples of pharmacopoeias are the Brazilian pharmacopoeia, the United States Pharmacopeia, the European pharmacopoeia and the international pharmacopoeia.

In a fifth object, the present invention discloses a hair treatment method comprising the application to the hair of a hair composition as defined in the first object.

In one embodiment, the hair treatment method comprises applying a hair formulation as defined above.

In one embodiment, 0.1 g to 5 g of formulation is applied to 1.0 g of hair in the hair treatment method. In one embodiment, 0.1 g to 1 g of formulation is applied to 1.0 g of hair. In one embodiment, 0.3 g to 0.5 g of formulation is applied to 1.0 g of hair.

In one embodiment, application of the composition is carried out for 0.5 minutes to 5 minutes. In one embodiment, application of the composition is performed for 0.5 minutes to 2 minutes. In one embodiment, application of the composition is performed for at least 1 minute.

In one embodiment, the composition is a shampoo. In this embodiment, the shampoo is applied to damp hair in an amount of 0.5 g of formulation per 1.0 g of hair. In one embodiment, the hair strands are gently massaged for 1 minute. In one embodiment, the strands are rinsed for 1 minute.

In one embodiment, the composition is a conditioner. In this embodiment, after rinsing the hair, an amount of 0.5 g of formulation (conditioner) is applied to 1.0 g of hair over the entire length of the strands, massaging and detangling the hair for 1 minute. Then the strands are rinsed for 1 minute.

In one embodiment, the composition is a leave-in. In that embodiment, the composition is applied to the dry hair strands in an amount of 0.3 g of formulation for 1.0 g of hair over the entire length of the strands.

In one embodiment, the hair composition or formulation is applied before, during and/or after hair discoloration processes. In one embodiment, the hair composition or formulation is applied during and/or after hair discoloration processes.

In one embodiment, the application is carried out during the discoloration process, with the application effect being called a protective effect or after a hair discoloration process, with this application effect being called a repairing effect. In one embodiment, the application takes place on hair that has not gone through hair discoloration processes. In one embodiment, the treatment method strengthens the hair. In one embodiment, the hair treatment method promotes a protective effect on the cuticular surface. In one embodiment, the hair treatment method increases hair brightness.

In one embodiment, the hair treatment method promotes a protective effect with a 5% to 15% increase in the maximum breaking strength of discolored hair. In one embodiment, the hair treatment method promotes a protective effect with a 7% to 10% increase in the maximum breaking stress of discolored hair. In one embodiment, the hair treatment method promotes repairing effect with a 5% to 25% increase in maximum breaking strength of discolored hair. In one embodiment, the hair treatment method promotes a repairing effect with an 8% to 20% increase in the maximum breaking strength of discolored hair.

In one embodiment, the hair treatment method promotes a protective effect with a 10% to 30% decrease in average combing strength. In one embodiment, the hair treatment method promotes a protective effect with a 15% to 25% decrease in average combing strength.

In one embodiment, the hair treatment method promotes a protective effect with an increase of 10% to 50% in the brightness unit. In one embodiment, the hair treatment method promotes a protective effect with a 20% to 40% increase in brightness unit. In one embodiment, the hair treatment method promotes a protective effect with a 25% to 35% increase in brightness unit.

In one embodiment, the hair treatment method promotes a protective effect on the alpha-keratin structure with an increase of 10% to 30% in enthalpy evaluated through DSC (Differential Scanning Calorimetry) measurements. In one embodiment, the hair treatment method promotes a protective effect on the alpha-keratin structure with a 15% to 25% increase in enthalpy evaluated through DSC (Differential Scanning Calorimetry) measurements. In one embodiment, the hair treatment method promotes a repairing effect on the alpha-keratin structure with an increase of 5% to 20% in the enthalpy evaluated through DSC (Differential Scanning Calorimetry) measurements. In one embodiment, the hair treatment method promotes a repairing effect on the alpha-keratin structure with a 10% to 15% increase in enthalpy evaluated through DSC (Differential Scanning Calorimetry) measurements.

The hair composition and/or formulation of the present patent application, comprising bifunctional α,β-unsaturated compounds in combination with ionic and non-ionic crosslinking agents, show superior and surprising results compared to other compositions used in the industry (figures 1 to 14). Furthermore, the test results prove the synergy of the composition, since the results obtained are much superior to those of the components alone, or even in compositions that do not present the combination of bifunctional α,β-unsaturated compounds and ionic and non-ionic crosslinking agents.

In the present invention, it is understood by:
Crosslinks: as used herein, the term "crosslinks" refers to crosslinks between the polypeptide chains of keratin, promoting an increase in resistance to deformation in the stretching of hair structures.
Ionic crosslinking agent (crosslink): as used herein, the term "ionic crosslinking agent" refers to ionically charged molecules capable of crosslinking keratin polypeptide chains.
Non-ionic crosslinking agents: as used herein, the term "non-ionic crosslinking agents" refers to uncharged molecules capable of increasing crosslinks between keratin polypeptide chains, promoting a synergistic association in increasing mechanical strength of the hair fiber.
Leave-in: as used herein, the term "Leave-in" refers to non-rinse-off hair products, styling products and conditioners.
Protective effect: as used herein, it is the effect of the compositions on the hair fibers when the product is applied during the discoloration process.
Repairing effect: as used herein, it is the effect of the compositions on the hair fibers when the product is applied after the discoloration process.
Bifunctional compound: as used herein, is a compound with at least two organic functions, i.e., two carboxylic groups, one amide group and one carboxylic group, two carboxylic groups and one amide group, three carboxylic groups, etc. Non-exhaustive examples of bifunctional compounds can be found in US2020315936.
Saccharide: is a common term in the prior art for carbohydrates. Examples of saccharides are sugars, starches and celluloses.

### Examples

The examples shown herein are intended only to exemplify one of the many ways to carry out the invention, but without limiting its scope.

### Example I - Hair Compositions

The hair compositions were prepared by mixing the ingredients according to table 1 below.

**Table 1. Composition of Bases A and B.**

| Component | Base A (% by mass) | Base B (% by mass) |
|---|---|---|
| Itaconic Acid | 5 - 25 | 5 - 20 |
| Arginine | 5 - 20 | 5 - 20 |
| Vegetable hydrolyzed rice protein | 1 - 10 | - |
| Disaccharides | 5 - 20 | - |
| Polyglyceryl-10 Laurate | 1 - 8 | 1 - 8 |
| Panthenol | - | 1 - 15 |
| Flaxseed Extract | - | 1 - 10 |
| Chia extract | - | 1 - 9 |
| Bis-Aminopropyl Diglycol Dimaleate | - | - |
| Phenoxyethanol | - | - |
| Sodium benzoate | - | - |
| Additives (Preservative) | 1.00 - 3.40 | 0.80 - 2.80 |
| Water (q.s. 100%) | q.s. 100% | q.s. 100% |

| | | |
|---|---|---|
| Benchmark 1: Water, Bis-Aminopropyl Diglycol Dimaleate, Phenoxyethanol, Sodium Benzoate. | | |

### Example II - Performance tests

In order to evaluate the effectiveness of the protective effect, tests were carried out to evaluate the mechanical properties (maximum tension), average combing strength, brightness, evaluation of the cuticular surface by scanning electron microscopy and protection of the crystalline structure of alpha-keratin by Differential Scanning Calorimetry (DSC) during 2 or 3 discoloration processes in hair samples, composed of a naive control group, a discolored control group, a discolored group with application of the benchmark 1 composition and a discolored group containing the present invention, being that the discoloration formulation presented 7.7% of the composition, and the composition could be Base A, Base B or Benchmark 1.

The hair samples consist of 100% natural hair strands with an average weight of 2 g per strand, which were subjected to the discoloration process.

The discoloration process consisted of preparing a discoloration mixture with 30 g of discoloration powder and 60 g of hydrogen peroxide 30 or 40 volumes, which was uniformly applied to bleach the strand of hair in the control group. For the test group, 7.5 g by mass of the hair composition of the present patent application or Benchmark 1 was added. This mixture was applied uniformly to the group hair strands wherein, after application, the strands were wrapped in aluminum foil and allowed to rest for 30 minutes.

After the time of action of the bleaching mixture, the strands were washed and rinsed abundantly with running water for 2 minutes, finally, the strands were dried using the dryer and this process was repeated two or three more times, totaling two or three discolorations.

The results allowed the evaluation that the hair compositions of the present patent application were successful in strengthening the hair samples when applied during the discoloration process, as can be seen in figures 1, 4-8, 13.

To evaluate the effectiveness of the repairing effect, tests of maximum tension were performed after 2 or 3 discoloration processes in hair samples, composed by a naive control group (without discoloration process), a discolored control group, a group discolored with application of the Benchmark 1 composition and a group containing the compositions of the present patent application after two or three discoloration processes, the composition being applied in a leave-in base containing 1% or 5% of the composition, this composition being the Base A or Base B, while Benchmark consisted of a ready-to-use cosmetic base (Benchmark 2 or Benchmark 3).

Benchmark 2: Water, Bis-Aminopropyl Diglycol Dimaleate, Propylene Glycol, Cetearyl Alcohol, Behentrimonium Methosulfate, Cetyl Alcohol, Phenoxyethanol, Glycerin, Hydroxyethyl Ethylcellulose, Stearamidopropyl Dimethylamine, Quaternium-91, Sodium Benzoate, Cetrimonium Methosulfate, Chloride Cetrimonium, Fragrance (Perfume), Polyquaternium-37, Tetrasodium EDTA, Butylphenyl Methylpropion, Etidronic Acid, Ascorbic Acid, Phytantriol, *Prunus amygdalus dulcis* (Sweet Almond) Oil, Tocopheryl Acetate, *Aloe barbadensis* Leaf Juice, Panthenol, *Simmondsia chinensis* (Jojoba) Seed Oil, Citric Acid, Potassium Sorbate.

Benchmark 3: Water, Bis-Aminopropyl Diglycol Dimaleate, Propylene Glycol, Cetearyl Alcohol, Behentrimonium Methosulfate, Cetyl Alcohol, Phenoxyethanol, Glycerin, Hydroxyethyl Ethylcellulose Stearamidopropyl Dimethylamine, Quaternium-91, Sodium Benzoate, Cetrimonium Methosulfate, Cetrimonium Chloride, Fragrance (Perfume), Polyquaternium-37, Tetrasodium EDTA, Butylphenyl Methylpropion, Etidronic Acid, Ascorbic Acid (Vitamin C), Phytantriol, Tocopheryl Acetate (Vitamin E), *Aloe barbadensis* Leaf Juice, Panthenol, *Simmondsia chinensis* Seed Oil (Jojoba), Citric Acid, Potassium Sorbate.

To evaluate the effectiveness of the synergistic repairing effect of the actives, maximum tension tests were performed, after 3 discoloration processes, in hair samples, composed of a naive control group (without discoloration process), a discolored control group, a discolored group with application of arginine, a discolored group with application of itaconic acid, a discolored group with application of arginine + itaconic acid and a group containing the Base B composition, with the compositions applied in a leave-in base containing 5% of the compositions. The leave-in base comprises: 3% - 5% cetostearyl alcohol 30/70; 0.8-2.5% cetyl alcohol 0.8-2.5%; 0.05% - 0.1% disodium EDTA; 0.8% - 2.5% CCTA cetyl trimethyl ammonium chloride; 0.25% - 1% preservatives and anti-microbial agents and water q.s. 100%.

**Table 2. Compositions of comparative solutions of itaconic acid (Itaconic acid), arginine (Arg) and itaconic acid + arginine.**

| Component | Arg aqueous solution (% by mass) | Itaconic acid aqueous solution (% by mass) | Arg+ltaconic acid aqueous solution (% by mass) |
|---|---|---|---|
| Itaconic acid | 5 - 20% | - | 5 - 20% |
| Arginine | - | 5-20 | 5 - 20% |
| Water | q.s. 100% | q.s. 100% | q.s. 100% |
| Basic aqueous solution | - | q.s. pH = 4.00 | - |
| Acid aqueous solution | q.s. pH = 4.00 | - | - |

For the evaluation of the repairing effect after basic straightening, the control strand was smoothed with a Sodium Hydroxide (NaOH) solution in a 1:2 ratio for 20 minutes, and after the time of action of the straightener, the strand was washed abundantly with water, and then the neutralizing shampoo was applied. The strand treated with the actives was smoothed with NaOH and after the straightening action time, the strands were treated with an aqueous solution containing 17% of the Base B or Benchmark 1 composition for 5 minutes. Afterwards, the strands were dried and tension measurements were taken.

For the evaluation of the restorative effect of the crystalline structure of alpha-keratin, Differential Scanning Calorimetry (DSC) was used through enthalpy measurements in hair samples composed of a discolored control group, a discolored group with application of composition of Benchmark 1 and a group containing the composition of the present patent application after three discoloration processes, the compositions being applied in a leave-in base containing 5% of the composition, this composition being Base A or Base B and Benchmark 1.

To test the efficiency of the hair composition after the discoloration process, hair strands (average weight of 2.0 g per strand) were subjected to a discoloration process.

A discoloration mixture was prepared with 30 g of discoloration powder and 60 g of hydrogen peroxide 30 volumes, and this mixture was evenly applied to the strands of hair. After application, the strands were wrapped in aluminum foil and kept for 30 minutes in the Suntest chamber at 40°C.

After the action time of the discoloration mixture, the strands were washed and rinsed abundantly with running water for 2 minutes; finally, the strands were dried using a dryer and the process was repeated twice more, totaling three discolorations.

After the discoloration process, a standardized amount of Leave-in was applied to the strands with or without (control) 1% or 5% of the hair composition of the present patent application on damp hair, and this composition may be the Base A or Base B or the actives making up Base B, while the benchmark consisted of a ready-to-use cosmetic base (Benchmark 2 or Benchmark 3). The strands of hair were gently massaged and left to rest for 30 minutes with the product in the hair. Then, they were rinsed for 1 minute.

This treatment was repeated twice, totaling three treatments. However, in the last treatment after the application of Leave-in, with or without (control) addition of 1% or 5% hair composition of the present patent application, this composition being able to be Base A or Base B, while the benchmark consisted of a cosmetic base ready for use (Benchmark 2 or Benchmark 3), the excess product was removed to carry out the resistance test. For the evaluation of the repairing effect by DSC, Benchmark 1 was used applied in a 5% leave-in.

The results allowed the evaluation that the hair compositions of the present patent application were successful in strengthening the discolored hair samples when applied after the discoloration process, as can be seen in Figures 2, 3, 9-12, and 14.

For both tests described above, the hair fibers were kept in a room with a controlled temperature of 22±2°C and relative humidity of 50±5% for at least 24 hours.

For the evaluation of mechanical properties, samples of hair fibers were randomly selected from each group. The samples referring to each treatment were subjected to the reading of the cross-sectional area of each fiber with the equipment FDAS-770 Dia-Stron Ltd and subsequently to the tensile test using the equipment MTT-680 -Automated Miniature Tensile Tester from Dia-Stron Ltd. Through the software of the equipment, the Maximum Tension parameter resisted by the fiber before rupture was evaluated, correlating the maximum force with the cross-sectional area of the capillary fiber.

For the evaluation of combability, the Texturometer Equipment TA.XT Plus by Stable Micro Systems LTD was used (with the hair combing rig accessory), with evaluations being carried out in 3 strands per group, with 20 measurements per strand, where the average strength (g) after 2 discoloration processes was evaluated. The measurement principle consists of passing the comb (probe) along the strand (vertically), registering the force required to comb/detangle the hair, along its length. For the test, two cycles (10 readings/cycle) were performed on each strand (03 strands per group).

For the evaluation of hair brightness, the SAMBA Hair System equipment from the company Bossa Nova Technologies, USA was used, being performed in three strands per group (6 measurements per strand), before and after treatment. The SAMBA system software calculates brightness simultaneously using different formulas. For this study, analysis data obtained by the Reich-Robbins formula were used, with measurements taken after 2 discoloration processes.

For the evaluation of the protection of the cuticular region, the Scanning Electron Microscope (SEM) was used, using 5 fibers per group, capturing 2 micrographs per fiber, with magnification between 400 and 1100 times. To capture the micrographs, initially, the hair fiber samples were metallized through the deposition of gold to cover the surface with Sputtering Denton Vacuum Desk II.

For the evaluation of the protective and repairing effect of the crystalline structure of alpha-keratin, Differential Scanning Calorimetry (DSC) was used, performing enthalpy measurements. Measurements were performed using approximately 2.0 mg of chopped hair from each group (five replicates) in an aluminum pan, partially closed using a DSC cell model 60 plus, Shimadzu Corporation^{®}, with a heating rate of 10°C/min (25-300°C), under dynamic N₂ atmosphere (flow rate of 50 mL/min). After acquiring the DSC curve of the hair sample, the enthalpy values were calculated and analyzed with the LabSolutions TA software (Version 1.01), Shimadzu Corporation^{®}.

### Example III - Performance tests against others hair products

Application tests of the compositions of this patent application after and during discoloration processes showed satisfactory results in increasing the strength of hair fibers.

The samples for each treatment were subjected to the reading of the cross-sectional area of each strand with the FDAS-770 Dia-Stron Ltd equipment and subsequently to the tensile test using the MTT-680 - Automated Miniature Tensile Tester from Dia-Stron Ltd. Through the software of the equipment, the Maximum Tension parameter resisted by the fiber before rupture was evaluated, correlating the maximum force with the diameter of the hair fiber.

The results obtained after application of the product in post-treatment discoloration tests were similar or superior to the Benchmark, as can be seen in Figures 3, 9-12, and 14.

The superior performance can be explained by the innovative mechanism of action promoted by the composition of the present patent application.

The observed superiority of the composition in relation to the Benchmark in tensile stress tests of post-treatment discolored hair can be explained by the more efficient crosslinking process of the polypeptide chains. These processes occur through 1,4-carbonyl addition reactions of amino group residues, present in the side chains of keratin amino acids, to itaconic acid (α,β-unsaturated compound) leading to the formation of an N-substituted lactam ring with carboxylate group, which has a strong interaction with arginine, hydrolyzed proteins and polysaccharides (crosslinking agents) leading to crosslinking of the keratin polypeptide chains.

### Discussion of the additional efficacy tests:

### Evaluation of Combability

The results of the Average Force measurements are shown in Figure 5. A reduction in the average force required for the comb to pass through the strand and untangle the hair indicates an improvement in combing. The Average Strength evaluated is inversely proportional to the ease of combing the hair. The lower the Average Strength, the easier it is to comb the hair, and therefore, the greater the effectiveness of the active.Through the results of the Average Force evaluation, it was verified that Base B presented a performance of hair fiber protection superior to Benchmark 1, where Base B presented a decrease of 19.5% in the average Force (g) in relation to the control (discolored), and 9.8% in relation to Benchmark 1. These results indicate a greater protective effect of Base B in the cuticular region of the fiber, since the active provided a significant reduction (p<0.05) in the force required to passing the probe (comb) over the strands in relation to the strands in the control group. A significant reduction in the force needed to comb the hair also reduces the chances of breaking or intensifying damage to the most fragile hair fibers because they have smaller diameters or have already suffered chemical or mechanical damage.

### Brightness Evaluation

The results of the Brightness unit measurements are shown in Figure 6. Through the results of the Brightness evaluation, it was verified that Base B presented a superior capillary fiber protection performance than Benchmark 1, and Base B presented a significant increase (p<0.001) of 29% in the brightness unit in relation to the control (discolored), and in relation to Benchmark 1. These results again indicate a greater protective effect of Base B in the cuticular region of the fiber, figure 6. Through figure 7, it is possible to observe that the significant increase in the value of the units of brightness, provided by the treatment with Base B, provides a visually perceptible increase in the brightness of the locks of hair.

### Evaluation of the cuticular surface (SEM)

The results of the micrographs, shown in Figure 8, showed that Base B had protective effects on the cuticular surface, showing less signs of cuticle abrasion, with less breakage and cuticle openings, with the possible formation of a barrier of protection without product buildup. The results corroborate with the results of improved combing, to show the effectiveness of the active in the cutillary region of the hair fibers, responsible for the sensory characteristics of the hair, such as softness, ease of combing and shine.

### Comparative evaluation of mechanical resistance with Benchmarks 2 and 3 - Base B applied at 5% in leave-in, after 3 discolorations.

The results are contained in Figure 9. It was verified that Base B applied at 5% in leave-in presented a performance of protection of the hair fiber superior to the benchmarks 2 and 3, being the value equal to 19.6% of significant increase (p<0.01) in tension compared to control (discolored) and compared to Benchmark 2 (p<0.05). Benchmark 2 showed a 1.4% increase in relation to the discolored group and Benchmark 3 a 7% increase in relation to the discolored group. The results of increased mechanical resistance of the hair fibers provided by Base 2 were 14 and 2.8 times greater than the results presented by Benchmarks 2 and 3, respectively. This reinforces the potential effectiveness of Base 2 in increasing hair fiber resistance and consequently reducing the risk of hair fiber breakage.

These results again corroborate the mechanism of action proposed for Base B, indicating that it promotes a higher density of crosslinks due to reactions with amino groups (-NH₂) and sulfhydryls or thiols (-SH), while for the Benchmark it is described that the mechanism of action occurs only via sulfhydryl groups (-SH). This difference is very important because the chemical treatments that are carried out on the hair, such as coloring and discoloration, transform the Sulfhydryl groups into sulphenic groups, and these are less reactive, making crosslinking less effective. Therefore, Base B reduces damage during chemical treatments, as well as restores hair resistance after chemical treatment.

### Comparative evaluation of resistance with Benchmark 3: Leave-in with 1% Base B, after 3 discolorations.

The results are contained in Figure 10. It was verified that Base B applied at 1% presented a performance of hair fiber protection similar to Benchmark 3, regarding the increase in tension in relation to the control (discolored), being the value equal to 8.3% for Base B and 8.9% for Benchmark 3, providing a significant increase (p<0.01) in relation to the discolored control group.

Therefore, surprising results were observed, because even at Base B concentrations below 5% the product showed significant performance, with a result similar to the Benchmark, evidencing its differentiated mechanism.

### Evaluation of the synergistic effect of the actives - Resistance evaluation - Leave-in with 5% Base B and leave-in with actives applied separately, after 3 discolorations.

The results are contained in Figure 11. The results demonstrated the synergistic effect of the actives that make up Base B, where it was possible to verify that there was a significant increase in resistance, in relation to the discolored strand with or without (p<0.001) the addition of the combination of associated itaconic acid + arginine (p<0.01), arginine (p<0.01) or itaconic acid (p<0.001). There was an increase of 9% in resistance, in relation to the discolored strand, when using Base B, while the evaluation of the association of itaconic acid + arginine and the actives tested separately, this increase reached increases of 4.5% using the association of itaconic acid and arginine, 3.9% using arginine and only 1.3% using itaconic acid. The hair fibers treated with Base B showed an increase in resistance two times greater than the fibers treated with an association of itaconic acid and arginine (bifunctional a,β-unsaturated compound and ionic crosslinking agent), which demonstrates the synergistic action between the components of Base B._Hair fibers treated with Base B did not show significant differences in resistance in relation to hair fibers without the discoloration process. This result again corroborates the mechanism of action proposed for Base B, indicating that it repairs the damage caused to the hair fibers, promoting greater density of crosslinking and recovering hair resistance.

### Evaluation of the mechanical resistance of strands straightened with alkaline straightener (Sodium Hydroxide)

The results are contained in Figure 12. There was an increase of 9.9% in the resistance of the strands treated with Base B in relation to the strands smoothed with Sodium Hydroxide (NaOH), while in the strands treated with Benchmark 1, there was an increase of only 4.6%. The results again corroborate the proposed mechanism of action for Base B, in which it promotes a greater density of reticulations. Hair subjected to straightening with NaOH can lead to the hydrolysis of peptide bonds leading to an increase in amino groups (-NH2) in the hair structure and these react with the Base B actives via Aza-Michael resulting in surprising values of protection when compared to the benchmark. That is, the composition is even more efficient in these types of treatment. This result proves the difference in mechanism between the products, where the better repair performance of the Base B fiber in relation to the Benchmark stands out. The result also proves that Base B actives can be used on damaged hair, due to different types of damage, such as chemical straightening and discoloration processes.

### Evaluation of the protective effect of the crystalline structure of alpha-keratin by DSC (Differential Scanning Calorimetry).

According to the results contained in Figure 13, it was possible to observe that the discolored strands with the addition of Base B had a significant increase of 21.2% in the enthalpy value in relation to the discolored control group (p<0.05), indicating that the presence of Base B provided an increase in the number of chemical bonds in the keratin structure, since the enthalpy value is directly proportional to the integrity of the protein content to the type and number of chemical bonds, leading to an effect of reduction of damage to the capillary keratin during the discoloration process. There was no statistical difference between the enthalpy values of the Base B and Benchmark 1 groups (p>0.05). As is known, the discoloration procedure is one of the most harmful chemical procedures and one of the most popular among consumers. This procedure aims to oxidize melanin (hair pigment), but during the process it also damages the protein of the hair shaft, affecting not only the hair color, but leading to the breaking of chemical bonds with loss of mechanical resistance of the hair (strength). Becoming important to protect the hair protein during this procedure, where the addition of Base B showed efficacy in protection compared to the Control group.

### Evaluation of the repairing effect of the crystalline structure of alpha-keratin by DSC (Differential Scanning Calorimetry).

According to the results contained in Figure 14, it was possible to observe that the discolored strands treated with the Leave-in + Base B 5% formulation had a significant increase of 12.1% in the enthalpy value in relation to the discolored Control group (p<0.05), indicating that the treatment with Leave-in + Base B 5% repaired the hair with a reticulation action, rebuilding the connections and interactions between capillary proteins (Multibond effect).

### Example VI - Formulations with several products

Said formulation has characteristics (stability and effectiveness over a wide pH range between 2 and 12 and solubility in water and water mixtures containing alcohol and polyols, and other polar solvents) that allow it to be adapted to different types of products for hair care, as in:

Conditioners, through compositions comprising 1% - 30% preferably 5% - 10% of the hair composition of the present patent application; 2.5% - 5% cetostearyl alcohol 30/70; 1% - 3% cetyl alcohol; 0.3% - 1.5% behenyl trimethyl ammonium chloride; 0.05% - 0.1% disodium EDTA; 0.5% - 3% cetyl trimethyl ammonium chloride (CCTA); 0.25% - 1% preservative and antimicrobial agents and water q.s. 100%.

Conditioners through compositions comprising 1% - 30% preferably 1% - 5% of the hair composition of the present patent application; with composition containing 1.5% - 3% hydroxyethylcellulose, 2.00 - 4.00% sodium astrocarium murumuruate, 2.00 - 4.00% *Orbignya speciosa* seed oil (and) *Astrocaryum murumuru* seed butter, 0.25 - 0.50% *Physalis angulata* extract (and) caprylic/capric triglycerides, 0.25 - 0.50% stearamidopropyl dimethylamine, 1.00 - 5.00% glycerin, 0.50 - 1.00% cetrimonium chloride, 0.50 - 1.50% polyquaternium-7, citric acid q.s. Ph 3.50 - 4.00, 1.00 - 3.00% xylitol phosphate (and) xylitol (and) water, 0.50 - 1.50% xylitol sesquicaprylate (and) caprylyl glycol and water q.s. 100%.

Leave-in by means of compositions comprising 1% - 30% preferably 5% - 10% of the hair composition of the present patent application; 3% - 5% cetostearyl alcohol 30/70; 0.8-2.5% cetyl alcohol 0.8-2.5%; 0.05% - 0.1% disodium EDTA; 0.8% - 2.5% CCTA cetyl trimethyl ammonium chloride; 0.25% - 1% preservatives and antimicrobial agents and water q.s. 100%.

Mask, by means of compositions comprising 1% - 30%, but preferably 5% - 10%, of the hair composition of the present patent application; 3% - 4% cetostearyl alcohol 30/70; 1% - 2% cetyl alcohol; 1% - 2% behenyl trimethyl ammonium chloride; 0.05% - 0.1%, disodium EDTA; 0.5% - 2% CCTA cetyl trimethyl ammonium chloride; 0.25% -1% preservative and antimicrobial agents and water q.s. 100%.

Serum, by means of compositions comprising 1% - 30%, but preferably 5% - 10%, of the hair composition of the present patent application; 3% - 4% cetostearyl alcohol 30/70; 1% - 2%, cetyl alcohol; 0.3% - 1% behenyl trimethyl ammonium chloride; 0.5% -1% babassu oil (and) *Astrocaryum murumuru* seed butter; 1% - 3% ethyl linoleate (and) ethyl oleate (and) *Crambe abyssinica* seed oil (and) *Helianthus annuus* (Sunflower) seed oil (and) *Persea gratissima* oil; 0.5% - 2% castor oil and propanediol esters; 0.25% - 1% preservative and antimicrobial agents and water q.s. 100%.

Shampoo: by means of compositions comprising 1% - 30%, but preferably 0.5% - 5%, of the hair composition of the present patent application; 1% - 10% cocoamidopropyl betaine; 20% - 30% sodium lauryl ether sulfate; 0.3% - 1% amide 90; 0.25% - 1% preservative and antimicrobial agents and water q.s. 100%.

Shampoo, through compositions comprising 0.1% -10% preferably 0.1% - 1% of the hair composition of the present patent application; with composition containing 0.50 - 1.00% polyquaternium-7, 0.05 - 0.10% disodium EDTA, 4.00 - 6.00% cocoamidopropyl betaine, 18 - 24.00% lauryl ether sulfate sodium, 1.00 - 3.00% lauryl glucoside, 1.00 - 2.00% sodium lauroyl sarcosinate, 3.00 - 5.00% cocamide dea, 0.10 - 0.50% xylitol sesquicaprylate, 0.10 - 0.50% fragrance, citric acid q.s., pH=6.50 - 7.00, 0.50 -1.00% d-panthenol, 1.00 - 3.00% of xylitol phosphate (and) xylitol (and) water, and water q.s. 100%.

Fluid spray emulsion, by means of compositions comprising 0.1% - 10% preferably 0.1% - 1% of the hair composition of the present patent application; with composition containing 20 - 25% C12-C15 alkyl benzoate, 0.50 - 2.00% castor oil and propanediol esters, 2.00 - 5.00% *Helianthus annuus* (sunflower) seed oil (and) *Zea mays* (maize) oil (and) *Sesamum indicum* (sesame) seed oil (and) *Macadamia integrifolia* seed oil (and) *Olea europaea* fruit oil (olive) fruit oil, 0.10 - 0.50% tocopheryl acetate, 20 - 30% water (and) cetearyl alcohol (and) mineral oil (and) polysorbate 60 (and) glycerin (and) cetyl acetate (and) acetylated lanolin alcohol, 0.50 -1.00% polyglycerol polyricinoleate, 1.00 - 2.00% glycerin, 0.25% - 0.50% xylitol sesquicaprylate, 0.50 - 1.00% cyclopentasiloxane, 1.00-3.00% xylitol phosphate (and) xylitol (and) water, water q.s. 100%.

Those skilled in the art will value the knowledge presented herein and will be able to reproduce the invention in the presented embodiments and in other variants and alternatives covered by the scope of the following claims.

## Claims

1. Hair composition **characterized in that** it comprises:
i. from 0.1% to 30% by mass of at least one bifunctional α,β-unsaturated compound and its derivatives;
ii. from 2% to 60% by mass of crosslinking agent, wherein:
a) from 1% to 40% by mass is a non-ionic crosslinking agent selected from the group consisting of polyols, saccharides, or a combination thereof;
b) from 1% to 20% by mass is an ionic crosslinking agent selected from the group consisting of hydrolyzed proteins, amino acids, or a combination thereof;
iii. water q.s. 100%.

2. Composition according to claim 1, **characterized in that** it further comprises 0.1% to 50% by mass of methionine sulfoxide.

3. Composition according to claim 1 or 2, **characterized in that** it comprises from 5% to 20% by mass of a bifunctional α,β-unsaturated compound selected from an α,β-unsaturated acid and/or dicarboxylic ester.

4. Composition according to any one of the preceding claims, **characterized in that** the non-ionic crosslinking agent comprises:
- from 1% to 15% by mass of polyol; and
- from 1% to 20% by mass of saccharides.

5. Composition according to any one of the preceding claims, **characterized in that** the ionic crosslinking agent comprises from 5% to 20% by mass of arginine.

6. Composition according to any one of the preceding claims, **characterized in that** it comprises from 1% to 15% by mass of additives.

7. Process for preparing a hair composition, as defined in any of the preceding claims, **characterized in that** it comprises the steps of:
a) mix from 0.1% to 30% by mass of at least one bifunctional α,β-unsaturated compound with 2% to 60% by mass of at least one crosslinking agent and water q.s. 100%.

8. Use of a hair composition, as defined in any one of claims 1 to 6, **characterized** for being in formulations of conditioners, leave-in, masks, serum, creams, shampoos, sprays, lotions or gels.

9. Hair formulation, **characterized in that** it comprises from 0.1% to 30% by mass of the hair composition as defined in any one of claims 1 to 6, and at least one cosmetically and/or pharmaceutically acceptable excipient.

10. Hair treatment method, **characterized in that** it comprises applying a hair composition, as defined in any one of claims 1 to 6, to the hair.

11. Hair treatment method, according to claim 10, **characterized in that** the application is before, during and/or after hair discoloration processes.
